# EUROPEAN PATENT SPECIFICATION

(11) **EP 1 342 410 B1**
(45) Date of publication and mention of the grant of the patent: **10.02.2010**
(21) Application number: 02028584.7
(22) Date of filing: 24.07.1997
(51) Int. Cl.: A01K 67/027, A61K 38/22, C12N 15/00, C12N 5/10, G01N 33/50, A61P 25/28

(54) **Use of a galanin agonist in the preparation of a medicament for improving memory and other cognitive functions**
Verwendung eines Galanin-agonisten in der Herstellung eines Medikaments zur Verbesserung des Gedächtnisses und anderer cognitiver Funktionen
Utilisation d'un agoniste de la galanine dans la préparation d'un médicament pour améliorer la mémoire et d'autres fonctions cognitives

(30) Priority: 24.07.1996 GB 9615551; 15.11.1996 GB 9623869
(43) Date of publication of application: 10.09.2003
(62) Divisional of application: 97932939.8
(73) Proprietor: Neurotargets Limited, Guildford, Surrey GU2 7YG (GB)
(72) Inventor: Wynick, David, University of Bristol,Dept.Medicine, Bristol, BS2 8HW (GB)
(74) Representative: Dean, John Paul

(56) References cited:
- WO-A-92/12997
- WO-A-92/15015
- WO-A-92/15681
- WO-A-92/20709
- WO-A-97/26853
- DATABASE WPI Section Ch, Week 9429 Derwent Publications Ltd., London, GB; Class B04, AN 94-238764 XP002045652 & JP 06 172387 A (AIBAITSU KK) 21 June 1994 (1994-06-21)
- BARTFAI T ET AL: "GALANIN AND GALANIN ANTAGONISTS MOLECULAR AND BIOCHEMICAL PERSPECTIVES." TRENDS PHARMACOL SCI 13 (8). 1992. 312-317, 1992, XP002045935
- UKAI M ET AL: "Effects of galanin on passive avoidance response, elevated plus-maze learning, and spontaneous alternation performance in mice." PEPTIDES (TARRYTOWN) 16 (7). 1995. 1283-1286, 1995, XP002045936
- WYNICK D ET AL: "GALANIN REGULATES BASAL AND OESTROGEN-STIMULATED LACTOTROPH FUNCTION." NATURE (LOND) 364 (6437). 1993. 529-532, 1993, XP002045651

## Description

This invention relates to galanin, including analogues thereof and its uses.

Galanin is a 29 amino acid neuropeptide which was first isolated from porcine intestine in 1983. Subsequently, the cDNA for galanin was cloned from a rat anterior pituitary library in 1987. Nucleotide and amino-acid sequence analysis suggests that galanin is unrelated to any of the other known families of regulatory peptides, and remains the only member of its family. The N-terminal portion of galanin is highly conserved between species, there being variation in the C-terminal portion.

Galanin has a widespread distribution in the peripheral and central nervous systems, gut and pancreas. It is found in highest levels in the median eminence of hypothalamus and in the pituitary

WO92/12997 (General Hospital Corporation), published in 1992, discloses the sequence of human galanin. There is a discussion of studies by other workers involving the administration of rat galanin or its N-terminal fragments to augment the effect of morphine and this patent application suggests that galanin can be expected to exhibit analgesic effects such that it may be administered alone or in combination with other analgesics. The application claims the use of galanin or its analogues in the treatment of pain and the use of galanin antagonists in the treatment of certain other conditions.

WO92/20709 (Astra AB) discloses a number of putative galanin antagonists. The antagonists which are described are all based on the first 12 amino acids of galanin followed by partial sequences of other peptides i.e. chimeric peptides. Some may be agonists, some antagonists and some may be both depending on the receptor subtype. The application discloses that the antagonists may be useful for treatment of insulin-, growth hormone-, acetyl choline-, dopamine-, Substance P-, Somatostatin-, and noradrenaline-related conditions including endocrinology, food intake, neurology and psychiatry, Alzheimer's type dementia, analgesia, intestinal disease. The application discloses the results of studies using some of the antagonists described therein on various effects such as galanin inhibition of glucose stimulated insulin release; galanin induced inhibition of scopolamine induced ACh hippocampal release; galanin induced facilitation of the flexor reflex; the displacement of bound iodinated galanin in membrane binding studies. There is a suggestion in the application that the antagonists may be indicated for analgesia but there is no disclosure in the application of results to this effect.

Approximately 2-4% of the Western population suffer from diabetes mellitus and, of those people, 10-15% suffer from chronic pain and numbness in their extremities-termed "painful neuropathy". Present techniques for management of painful neuropathy are inadequate.

Alzheimer's disease is a major cause of morbidity worldwide the disease being characterised by loss of memory and personality changes. At an anatomical level there is a major decrease in the number of cholinergic nerves in the basal forebrain and hippocampus, which are the main area of the brain thought to process and store memories. Previous work has shown that galanin is also expressed in these hippocampal nerves and the levels of galanin are two fold elevated in the brains of patients with Alzheimer's disease.

The invention relates to a method of improving memory, enhancing memory and improving cognitive function, comprising administering a galanin agonist to a subject. Advantageously, such treatment may be used in the treatment of restoring memory after injury, trauma or in Alzheimer's disease.

The present invention provides the use of a galanin agonist in the preparation of a medicament for the treatment of Alzheimer's disease and related diseases and conditions, and in enhancing memory and cognitive function.

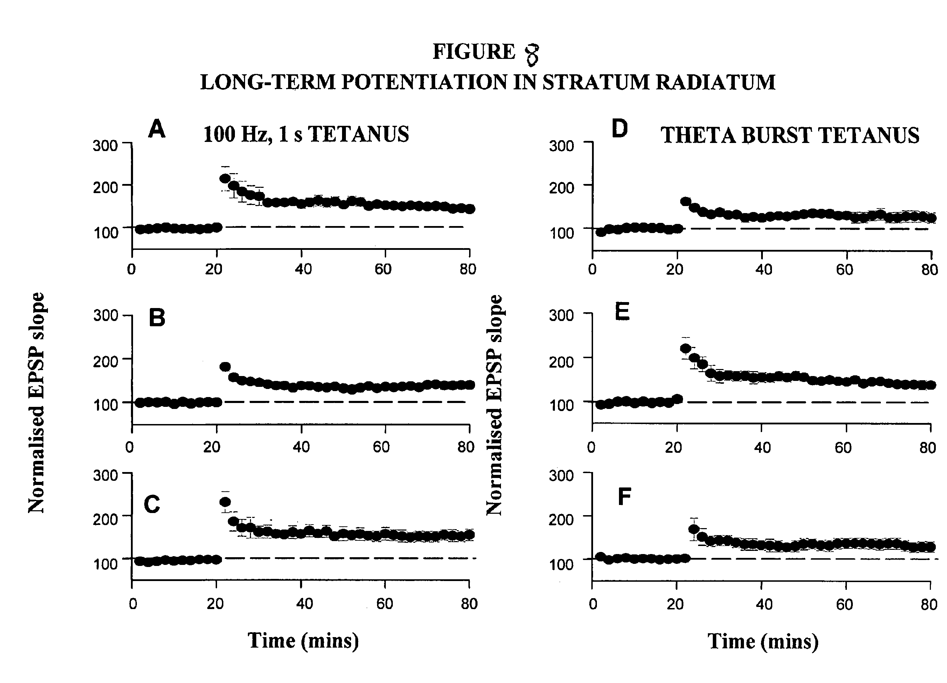

Embodiments of the invention will now be described, by way of example only, with reference to the accompanying drawings Figures 1 to 9 in which:
Fig. 1 illustrates the genomic structure of mouse galanin;
Fig. 2 illustrates the targeting vector used in producing the rodent of the invention;
Fig. 3 illustrates the specific recombination event in the production of the rodent in accordance with the invention;
Fig. 4 illustrates the genotype of the progeny determined using Southern blotting and by PCR demonstrating identical results from the same litter derived from a mating of two heterozygote animals;
Fig. 5 illustrates the effect of galanin inactivation on short term regeneration of sensory neurons;
Fig. 6 illustrates the effect of galanin inactivation on long term regeneration of sensory neurons;
Fig 7 illustrates expression of an exon 6-specific riboprobe to study the distribution of galaninergic neurons in the brain and dorsal root ganglion of wildtype and mutant mice;
Fig 8 illustrates the effects of galanin inactivation on the generation of long term potentiation in the stratum radiatum area of the hippocampus; and
Fig 9 illustrates the effects of galanin inactivation on the generation of long term potentiation in the stratum oriens area of the hippocampus.

To generate a mouse knockout, that is the introduction into the mouse genome of either a loss- or gain-of-function mutation of a specific gene locus (according to the procedure described in Kuehn, M. R. et al Nature. 1987; 326: 295-8; Thomas, K. R. and Capecchi, M. R. Nature. 1986; 324: 34-8), entails a number of steps:- (1) the cloning of the mouse genomic locus of interest; (2) the construction of a targeting vector such that the locus/gene of interest is modified to inactivate or alter its structure and function in some way; (3) introduction of the targeting vector into an embryonic stem cell library and selection and identification of single cell clones in whom the appropriate correct targeting event has taken place and in whom the normal chromosomal number is unchanged; and (4) introduction of such clones into 3.5 day old blastocysts and the resulting chimeric mice mated to wild types of the opposite sex. The resulting offspring demonstrated to carry the mutation are thus heterozygotes and, by appropriate mating, homozygotes for the introduced mutation are bred.

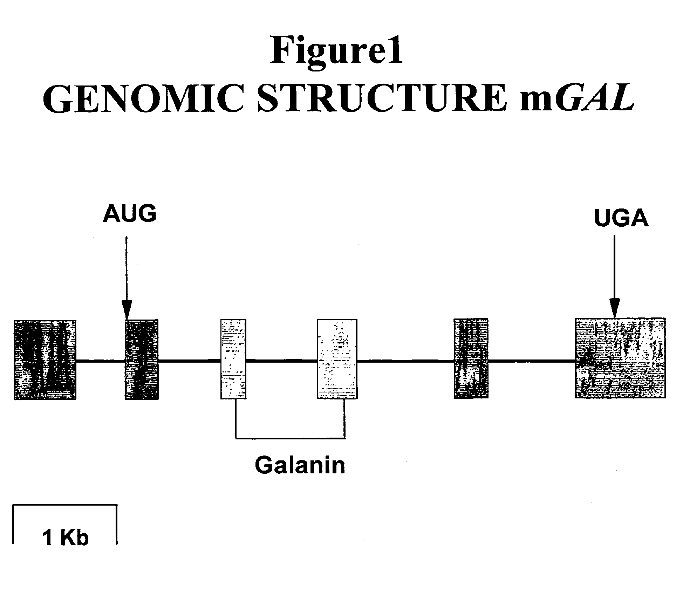

As a first step the murine *galanin* gene was cloned. A mouse genomic library (Ehrich, E. et al Gene. 1987; 57: 229-37) was screened using the full length rat *galanin* cDNA as a probe under high stringency. Two cosmid clones were identified spanning 60Kb around the galanin locus. Using 5' and 3' probes from the rat cDNA a 14 Kb region of DNA containing the entire gene was subcloned and partially sequenced. From the genomic sequence, primers were designed complementary to untranslated exonic regions of the gene. A 630bp fragment was generated by RT-PCR (Kit supplied by INVITROGEN BV, The Netherlands) using adult female whole brain as a source of mRNA. Subsequent sequencing of this fragment demonstrated that mouse and rat galanin are 100% identical at the protein level and 94.8% at the nucleotide level. The genomic structure of the mouse gene (Fig. 1) is identical to that of the rat gene. The gene spans 4.8Kb and consists of six exons. The translation start site (AUG) starts at the first base of exon two, the coding region for galanin extends across exons three and four with the stop codon (UGA) in the middle of exon six.

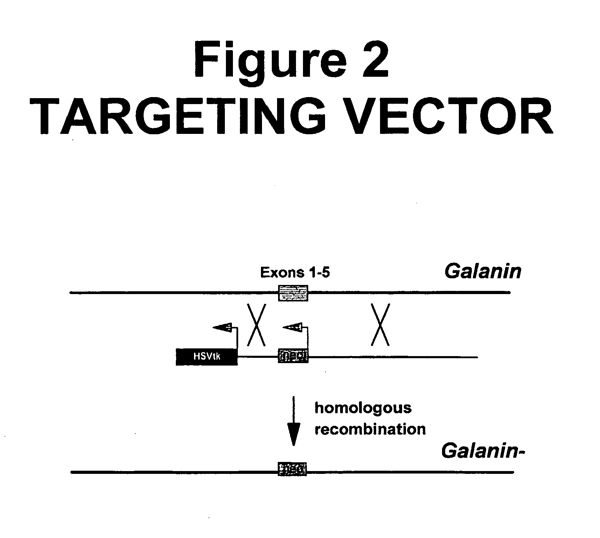

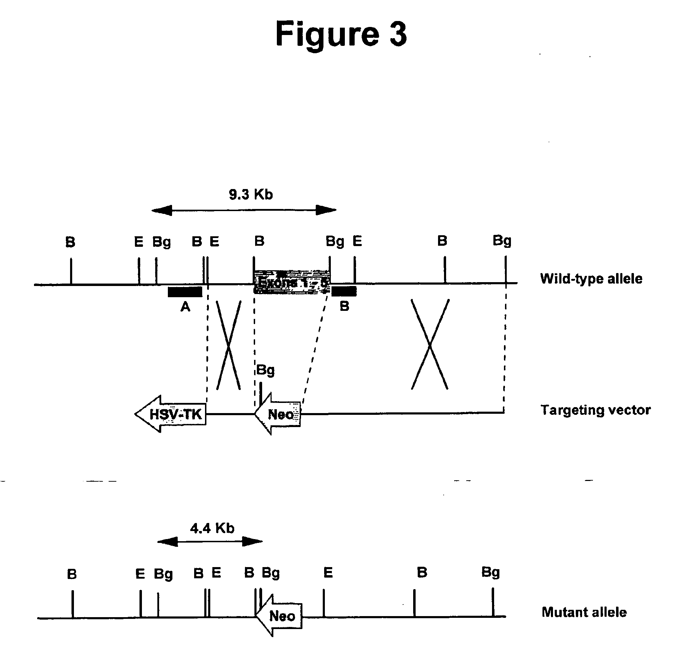

Using the 14Kb subclone described above, a positive/negative selection targeting vector was constructed (Fig. 2). The mutation introduced removes the first five exons containing the entire coding region of the galanin peptide (Fig. 3).

In Fig. 3: A and B are the sites of the external probes used to screen the ES cells for the appropriate integration of the construct.
Neo = neomycin resistance gene
HSV-TK= herpes simplex virus thymidine kinase gene
B *= Bam*HI
E=*EcoR*I
X=*Xho*I
Bg=*Bgl*II

In particular, the targeting vector removes a 3.2Kb stretch of DNA and thus removes the first 5 exons of the galanin gene. The exact sites flanking the stretch of DNA removed are 5' - the *BamH*I site 10bp downstream from the transcriptional start site and the 3' site is the *Bgl*II site in the middle of intron 5. These sites are indicated with asterisks in Fig. 3. Other sites that could be used are the same 5' site and a differing 3' *Xho*I site in intron 4 which would remove only 2.9Kb of DNA and thus remove only first 4 exons.

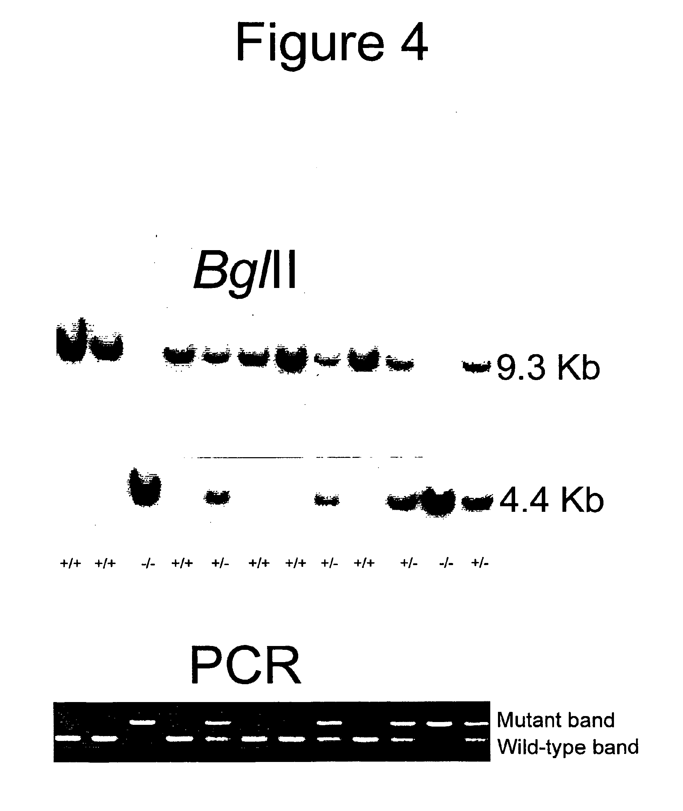

This vector was linearised and electroporated into the E14 embryonic stem-cell (ES) line (Hooper, M. et al Nature. 1987; 326: 292-5). Restriction mapping of the wildtype locus with *Bgl*II generates a 9.3Kb fragment when probed with a 5' external probe (marked A, Fig 3), whilst the correctly targeted locus generates a 4.4 Kb fragment. In total, 9 clones were identified in which one allele of the galanin gene was correctly targeted by homologous recombination among 209 double resistant colonies yielding a targeting frequency of 4.3%. These nine clones were karyotyped, confirming euploidy, and injected into 3.5 day old blastocysts from C57BL/6 mice. Germ line transmission of the disrupted galanin locus was obtained from three separate ES cell clones. Genotype of the progeny was determined using Southern blotting and by PCR (Fig 4 demonstrates identical results obtained by Southern blotting and PCR screening on the same litter derived from a mating of two heterozygotes). The mutation has been bred to homozygosity on the in-bred 129sv strain and all data presented is from mice on this background.
1. Results of genotype analysis of live births are in the expected ratio predicted by Mendelian genetics and the sex ratio is 1:1. Galanin levels were measured by radioimmunoassay and immunocytochemistry in areas previously demonstrated to express galanin at high levels and include brain, pituitary, spinal cord, dorsal root ganglion, stomach, small intestine and uterus. Galanin levels in heterozygotes for the deletion were 50% of wild type controls whilst Galanin levels in the homozygotes for the deletion were undetectable in all cases.

A comparision of levels of galanin expression between wild type, heterozygote and mutant mice in several body tissues is shown in Table 1.

**Table 1**

| **Genotype** | **Cortex** | **Hypothalamus** | **Anterior Pituitary** | **Stomach** | **Duodenum** | **Ileum** |
|---|---|---|---|---|---|---|
| +/+ | 5.78±0.3 3 | 110.34±7.81 | 0.42±0.07 | 27.46±1.91 | 122.90±11.60 | 267.43±1 3.46 |
| +/- | 2.91±0.2 1 | 53.82±3.76 | 0.21±0.04 | 13.8±0.83 | 68.36±5.67 | 125.87±7 .55 |
| -/- | UD | UD | UD | UD | UD | UD |

All values are mean galanin-LI pmol/g ± SEM, other than the female anterior pituitary which is expressed as pmol/gland ± SEM. UD=Undetectable

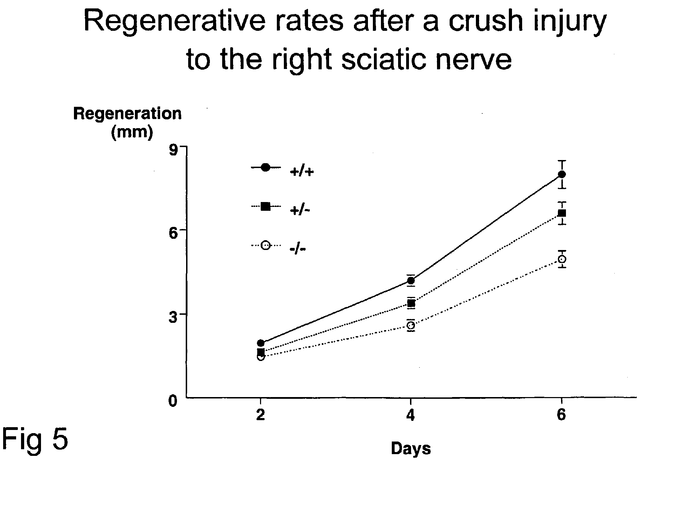

It will be seen that galanin was not detected in any of the tissues tested in the homozygous mutant mouse, and decreased by 50% in the heterozygous mutant mouse.
2. The regenerative abilities of sensory axons in the sciatic nerve were directly measured by the pinch test (Danielsen, N., Kerns, J.M., Holmquist, B., Zhao, Q., Lundborg, G. & Kanje, M. Brain Res. 681, 105-108 1995). Following nerve crush, sensory axons regenerate into the distal nerve and can be stimulated by a subsequent nerve pinch, which elicits a reflex abdominal motor response. The foremost regenerating axons are located by pinching consecutive segments of the nerve in a distal to proximal direction until a reflex is observed and the distance from the nerve crush can be calculated. In a comparative example regeneration showed a statistically significant reduction of 30-40% in homozygotes compared to wild type mice at 2, 4 and 6 days after nerve crush (Fig. 5). Regeneration was intermediate in heterozygous mice but was still significantly different from wild type animals.

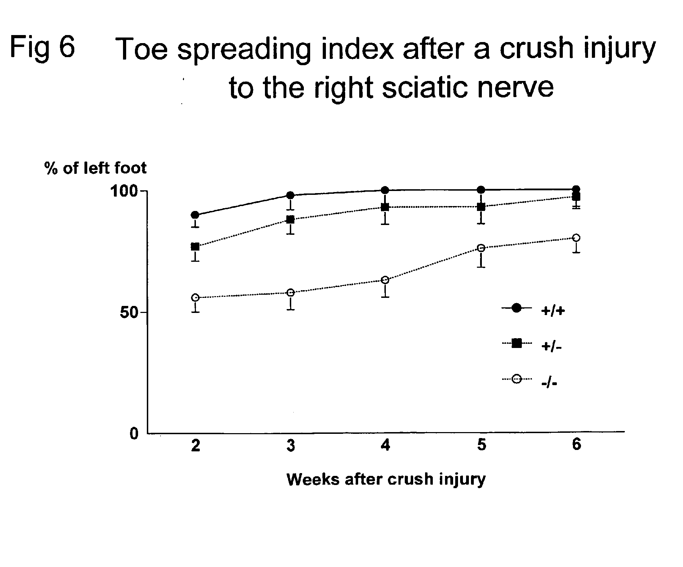

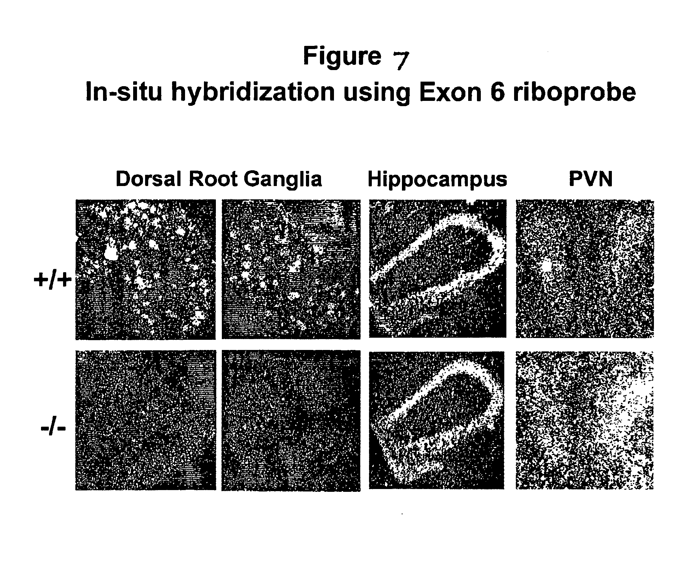

To test whether the reduced rate of regeneration in galanin-deficient mice affects functional recovery after a crush injury, we tested a behavioural correlate of regeneration using the toe spreading index (Hoogeveen, J.F., Van Der Kracht, A.H., Wondergem, J., Gonzalez Gonzalez, D. & Haveman, J. Neurotoxicology. 14, 1-7 1993). Rodents spread the toes on their hind feet upon contact with a solid surface, a response which requires sensory innervation. Toe spreading is, therefore, lost after axotomy until sensory axon re-innervation occurs. The toe spreading distance was measured for 6 weeks after unilateral right sciatic nerve crush and compared to the intact contralateral (left) foot. Whilst toe spreading in wild-type mice returned to normal within 3 weeks of sciatic nerve crush, functional regeneration was still incomplete at six weeks in the mutant mice (Fig 6).
3. The decreased regeneration and autotomy in the galanin-deficient mice might be related to the death of neurons following axotomy, especially those neurons which would normally express galanin after injury. In a further comparative example, to test whether galanin is essential for the survival of neurons during development, we studied the distribution of galaninergic neurons in wild type and mutant mice. Since we were unable to visualise the galaninergic neurons in the mutant animals at the protein level we studied expression of the mRNA using a riboprobe specific for exon six (marked B, see Figure 3). In order to confirm the survival of other populations of galanin expressing neurons, the exon 6-specific riboprobe was used to visualise galaninergic neurons in the hippocampus and the paraventricular nucleus of the hypothalamus of adult wild-type and mutant mice (Fig 7). No differences in expression were observed between the groups suggesting that neuronal development is normal in these animals and not galanin dependent.

We went on to use the exon 6-specific riboprobe to study the distribution of galaninergic neurons in the DRG two weeks after sciatic nerve axotomy. A marked up-regulation in the levels and number of cells expressing galanin was observed in the DRG neurons of wild type mice Fig 7). However, there was no expression in the homozygous galanin- deficient mice, suggesting that galanin is required for these cells to survive axotomy.

These results relating to regeneration and cell survival are particularly significant in that the results indicate that galanin gene is the first gene to affect regeneration of the peripheral nervous system.

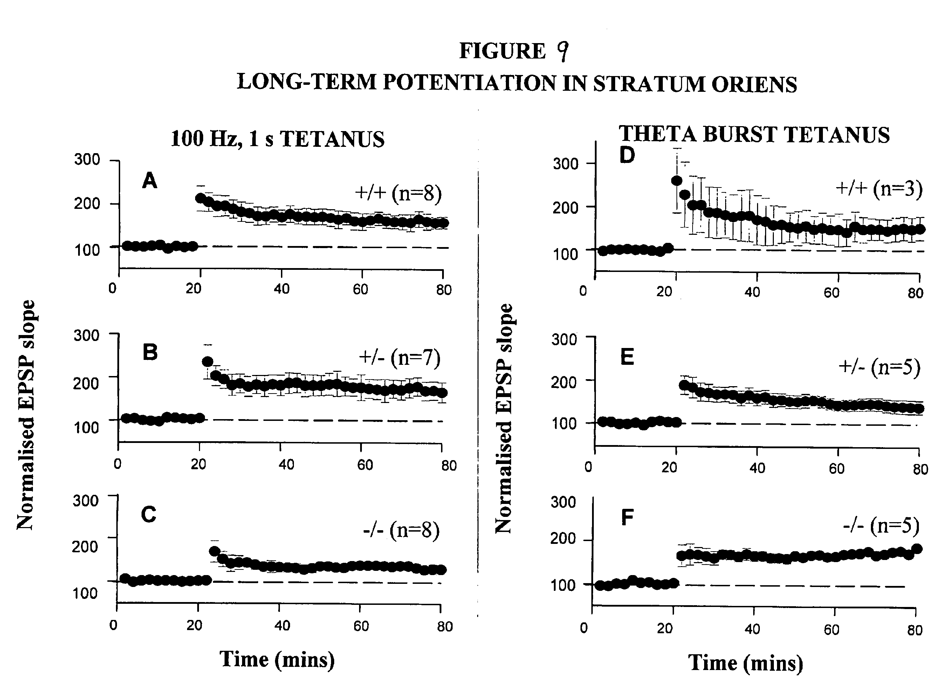

Accordingly, the invention contemplates the use of a galanin agonist in the treatment of peripheral sensory neuropathy resulting, for example, from diabetes mellitus or trauma (such as that caused by traffic accidents).
4. Galanin has been implicated in the aetiology of Alzheimer's disease. Hippocampal galanin expression is increased in cholinergic neurones as acetylcholine and choline acetyl transferase (ChAT) levels fall. Administration of galanin decreases learning behaviour in a number of mouse models, the converse is also true when galanin antagonists are infused. We measured long term potentiation (LTP) in wild type and mutant mice. LTP is an electrophysiological test where specific nerves in the hippocampus are stimulated by an electric shock: Davies CH, Collingridge GL. J. Physiol. Lond. 1996;496: 451-470; Davies CH, Starkey SJ, Pozza MF, Collingridge GL. GABA Nature 1991;349:609-611. This procedure is done *in-vitro* using brain slices from recently killed animals. Results show that LTP is decreased by 50% in the stratum oriens at the 80 minute time point in the mutants compared to wild-type mice (Fig 9 A vs C). In contrast no difference was found in LTP measured in the stratum radiatum. Galanin is found at high levels in the stratum oriens but NOT in the stratum radiatum. Our data, thus far, demonstrates a decrease in LTP in the mutants implying a decrease in memory and cognition - tests to assess these function are being conducted. These data show that a galanin agonist is useful in the treatment of Alzheimer's disease and associated memory loss with an enhancement in memory and cognition.

## Claims

1. The use of a galanin agonist in the preparation of a medicament for improving memory and other cognitive functions.

2. The use according to claim 1 for the treatment of Alzheimer's disease.

## Patentansprüche

1. Verwendung eines Galanin-Agonisten zur Herstellung eines Medikamentes zur Verbesserung der Gedächtnisleistung und anderer kognitiver Funktionen.

2. Die Verwendung nach Anspruch 1 zur Behandlung der Alzheimerschen Erkrankung.

## Revendications

1. Utilisation d'un agoniste de la galanine dans la préparation d'un médicament pour améliorer la mémoire et d'autres fonctions cognitives.

2. Utilisation selon la revendication 1 pour le traitement de la maladie d'Alzheimer.
